# EUROPEAN PATENT APPLICATION

(11) **EP 1 925 305 A1**
(43) Date of publication of application: **28.05.2008**
(21) Application number: 06122746.8
(22) Date of filing: 23.10.2006
(51) Int. Cl.: A61K 31/423, A61K 31/4168, A61K 31/55, A61P 25/26

(54) **Ih channel inhibitors for the promotion of wakefulness**

(71) Applicant: N.V. Organon, 5349 AB Oss (NL)
(72) Inventor: The designation of the inventor has not yet been filed
(74) Representative: Van den Broek, Ludovicus A.G.M.

(57) **Abstract**

The present invention relates to the use of an Iₕ channel inhibitor for the manufacture of a medicament for the promotion of wakefulness in a subject in need thereof, in particular, wherein the subject is suffering from excessive sleepiness which may result from a sleep disorder.

## Description

The present invention relates to a method of treatment or prevention of drowsiness and promotion of wakefulness, especially in humans, comprising administering an effective amount of a medicine to a subject in need thereof. The method also relates to a medicinal use of Iₕ channel inhibitors.

Patients with a variety of sleep disorders can suffer from excessive drowsiness that further impacts on their daily life. As a remedy, drugs have been used to increase or sustain levels of wakefulness in order to help maintain or improve levels of attention, vigilance and other aspects of cognitive function. For example, amphetamines, long associated for their ability to act as performance enhancing drugs, have also been used to improve levels of wakefulness. Although amphetamines can have a beneficial effect in low dosage treatment regimes, their use is not ideal since following the use of the drug can come long periods of sleep often followed by hunger and depression which can give rise to a need for further dosage. With the use of amphetamines to promote increased alertness therefore there is the risk of addiction. Additionally molecules such as amphetamines are known to stimulate motor systems and affect cardiovascular function. Both effects should be considered as unwanted side-effects that need to be avoided.

Efforts continue to discover further drugs which can treat or prevent drowsiness without the risk of side-effects. Modafinil, for example, is one example of a drug which can improve wakefulness in patients without interfering with normal sleep. There remains a need, however, for further treatment regimes which are both safe and effective.

The present invention provides to a method of promoting wakefulness, comprising administering an effective amount of an Iₕ channel inhibitor to a subject in need thereof. The present invention therefore provides use of an Iₕ channel inhibitor for the manufacture of a medicament for the promotion of wakefulness in a subject in need thereof.

The hyperpolarisation activated cation current (Iₕ), also known as queer current (I_{q}) or funny current (I_{f}) is produced by hyperpolarisation cyclic nucleotide (HCN) channels which are found in a variety of excitable cells, including neurones, cardiac pacemaker cells and photoreceptors. There are four subtypes of HCN channel (HCN 1-4) which differ in their biophysical properties, sensitivity to cyclic AMP and distribution. The best known function of Iₕ is to control heart rate and rhythm by acting as a 'pacemaker current' in the sinoatrial (SA) node. Iₕ is activated during membrane hyperpolarisation following the termination of an action potential and provides an inward Na⁺ current that slowly depolarises the plasma membrane. Sympathetic stimulation of SA node cells raises cyclic AMP levels and increases Iₕ, thus accelerating diastolic depolarisation and heart rate. In neurons Iₕ fulfills diverse functions, including generation of pacemaker potentials (neuronal pacemaking), determination of resting potential, transduction of sour taste, dendritic integration, control of synaptic transmission and plasticity.

Several structurally unrelated Iₕ channel inhibitors are known in the art. The benzazepin-2-one, zatebradine hydrochloride *i.e.,* 1,3,4,5-tetrahydro-7,8-dimethoxy-3-[3-[[2-(3,4-dimethoxyphenyl)ethyl]methylimino]propyl]-*2H*-3-benzazepin-2-one hydrochloride is disclosed in EP 65229. The related compound ivabradine hydrochloride, *i.e.,* (+)-3-[3-[N-[4,5-dimethoxybenzocyclobutan-1(*S*)-ylmethyl]-*N*-methylamino]propyl]-7,8-dimethoxy-2,3,4,5-tetrahydro-*1H*-3-benzazepin-2-one hydrochloride is disclosed in EP 534859. ZD 7288, *i.e.,* 4-(*N*-ethyl-*N*-phenylamino)-1,2-dimethyl-6-(methylamino)pyrimidinium chloride is a bradycardic agent disclosed in GB 2230527. Alinidine *i.e.,* 2-[N-allyl-N-(2,6-dichlorophenyl)amino]-2-imidazoline is disclosed in DE 1958201. Org 34167, *i.e.,* (S)-2-(1,2-benzisoxazol-3-yl)-α-2-propenylbenzenemethanamine hydrochloride is disclosed in WO 97/40027. Certain other Iₕ channel inhibitors are disclosed in WO 99/18941.

Iₕ channel inhibitors have been indicated to be useful as bradycardic agents (see Neuroscience, 1994, 59(2), 363-73). More recently Iₕ channel modulators have been indicated to be useful in the treatment of a range of other therapeutic indications including anxiety, post traumatic stress disorder and acute stress disorder, attention deficit disorders, eating disorders, personality disorders, schizophrenia, substance-related disorders and sexual function disorders (see WO 99/18941). Iₕ channel inhibitors are also implicated in the treatment of neuropathic pain (see J. Yagi et al., Proceedings of the 9th World Congress on Pain, Progress in Pain Research and Management, 2000, vol. 16, pages 109-116).

WO 99/18941 discloses that Iₕ channel modulators have application in sleep disorders in general and more specifically in narcolepsy-cataplexy syndrome, a disorder characterised by episodes of sudden loss of muscle tone with accompanying sleepiness. Whilst Iₕ channel modulators were indicated to be useful in sleep disorders in general, there is no suggestion of the particular efficacy of Iₕ channel inhibitors in the promotion of wakefulness, which has now been found. Thus, it has now been found that Iₕ channel inhibitors are particularly effective in the treatment of the symptoms of sleepiness. This is different from the previously disclosed use of Iₕ channel modulators for treatment of the sleep disorder itself. The symptoms of sleepiness may be secondary or unrelated to a particular sleep disorder. Furthermore modafanil, a compound known to be useful in improving wakefulness was shown not to be an Iₕ channel inhibitor.

Wakefulness, as used herein, denotes a state in which there is enhanced potential for sensitivity and an efficient responsiveness to external stimuli. Improved wakefulness results in less drowsiness and greater vigilance. Drowsiness refers to a feeling of abnormal sleepiness, often with a tendency to fall asleep in inappropriate situations or at inappropriate times. Vigilance refers to a state of being awake, alert and watchful.

In one embodiment of the present invention is use in a subject showing the symptoms of excessive sleepiness. Excessive sleepiness, as used herein, denotes a greater than normal suspension of sensorimotor interaction with the environment, usually associated with recumbency and immobility. Excessive daytime sleepiness is a specific example.

In a further embodiment of the present invention, the excessive sleepiness arises from sleep deprivation, for example, as a result of a sleep disorder. A sleep disorder refers to a neurological disorder marked by a sudden, recurrent and uncontrollable compulsion to sleep. The skilled person will appreciate that such a sleep disorder can include a primary sleep disorder, for example, a dyssomnia such as primary insomnia, a breathing related sleep-disorder, a circadian rhythm sleep disorder, a parasomnia, such as nightmare disorder or sleepwalking disorder. The excessive sleepiness can alternatively result from a sleep disorder related to another medical condition, for example, a sleep disorder related to a mental disorder such as anxiety or a sleep disorder related to conditions such as hypothyroidism, acromegaly or kyphoscoliosis. Further, the excessive sleepiness can result from substance induced sleep-disorder, for example, excessive sleepiness induced following administration of opiates, such as morphine, or following ingestion of substances such as alcohol or barbiturates. In a further embodiment the sleep disorder is obstructive sleep apnea/hypopnea syndrome, insomnia or shift work sleep disorder.

In a further embodiment of the present invention is use in a specific patient population, for example, use in the elderly or use in patients with loss of circadian control, such as, use in military personnel.

In a further embodiment of the present invention is the use of an Iₕ channel inhibitor selected from:
1,3,4,5-tetrahydro-7,8-dimethoxy-3-[3-[[2-(3,4-dimethoxyphenyl)ethyl]methylimino]propyl]-2H-3-benzazepin-2-one;
(+)-3-[3-[*N*-[4,5-dimethoxybenzocyclobutan-1(S)-ylmethyl]-*N*-methylamino]propyl]-7,8-dimethoxy-2,3,4,5-tetrahydro-*1H*-3-benzazepin-2-one;
2-[*N*-allyl-*N*-(2,6-dichlorophenyl)amino]-2-imidazoline and
(S)-2-(1,2-benzisoxazol-3-yl)-α-2-propenylbenzenemethanamine or a pharmaceutically acceptable salt or solvate thereof. These Iₕ channel inhibitors are all centrally acting.

The Iₕ channel inhibitor for the use according to the present invention can be combined with other drugs, for example, with stimulants, hypnotics or antidepressants.

Pharmaceutical compositions for the use in accordance with the present invention can be prepared utilising standard techniques in the art of pharmaceutical sciences. The compositions can be used for humans in a dosage of 0.001-40 mg per kg body weight, preferably in a dosage of 0.01-20 mg per kg body weight, whereby the optimum dosage can be determined according to factors such as route of administration, desired duration of action, type of formulation (extended release versus immediate release) type of patient, type of compound required, efficacy of the compound and other physical characteristics of the recipient of the treatment, such co-morbidity of other diseases, liver metabolism capacity, *etc*. In a further embodiment, a composition comprising (S)-2-(1,2-benzisoxazol-3-yl)-α-2-propenylbenzenemethanamine is administered at a dosage of 0.001-2 mg per kg body weight.

In a further embodiment, the dosage regime is administered at the beginning of the day. In a further embodiment, the dosage regime is administered at the start of an individual's professional activity wherein alertness needs to be maintained or enhanced.

Iₕ channel inhibition and methods how to determine such a biological effect can be realised according to known techniques in the biochemistry of HCN channels. A specific method is described in the example below, on which basis a criterion pIC₅₀ value of at least 6.0, or preferably 6.5, or even better 7.0 can be derived for clarity of the meaning of the term Iₕ channel inhibitor.

Wakefulness can be determined by the maintenance of wakefulness test (MWT). In this test the subject is placed in a quiet, dimly lit room and instructed to remain awake. The protocol is then repeated several times during the day. Sleep latency (the amount of time before falling asleep) is measured on each trial and is used as an index of the individual's ability to maintain wakefulness.

### References

i. Ruigt, G.S., Van Proosdij, J.N. and Van Wezenbeek, L.A., A large scale, high resolution, automated system for rat sleep staging II, validation and application, Electroencephalogr, Clin. Neurophysiol., 1989, 73, 64-71.
ii. Ruigt, G.S., Van Proosdij, J.N., Vqan Delft, A.M.L., A large scale, high resolution, automated system for rat sleep staging I, methodology and technical aspects, Electroencephalogr. Clin. Neurophysiol., 1989, 73, 52-63.

### Example 1 - Determination of Iₕ channel inhibition

**Methods:** HEK.hHCN1.2H10 cells growing in culture were dissociated using CDS to lift the cells. They were suspended in high K external solution and used in the assay at a density of 2x10⁶ cells.ml⁻¹. Two separate runs were performed with voltage steps to -80mV and 120mV for 1s; the holding potential in both cases was -40mV. The amplitude of the time-dependent current (assumed to be all hHCN1) was measured as the difference between the current recorded immediately after the capacity transient on stepping to the test voltage and the current measured after it had reached a steady state amplitude (PeakAmp metric). The amount of inhibition was assessed by calculating the ratio of Post Compound PeakAmp/Pre Compound PeakAmp. Thus a ratio of 1 would indicate no inhibition and 0 would indicate 100% inhibition. Compounds were tested at concentrations between 0.152µM and 333.3µM, and a dose-response curve constructed. The IC₅₀ value was obtained (the concentration causing half maximal inhibition) and the pIC₅₀ calculated (the negative logarithm of the molar concentration causing half-maximal inhibition).

### Results:

| | IC50 µM -80 mV | IC50 µM -120 mV |
|---|---|---|
| Org 34167 | 3.2 | 8.6 |

### Example 2 - The effect of Org 34167 on sleep-wake organisation in rats

**Methods:** Sleep-wake organization was evaluated in instrumented male rats held on a 12:12 light-dark cycle. Classification of sleep-wake states was measured using an automated system (ACSO, Automatic Classification of Sleep Organization) that records and processes EEG (cortical), EMG (neck muscle) and movement signals. Subjects were assigned to placebo or Org 34167 treatment groups and signals were recorded on two successive days. The first recording session provided a baseline recording following treatment with placebo only. This baseline day was used to extract a sleep classification algorithm for each individual subject. On the second day recording was started immediately after administration of placebo or Org 34167. Treatments were administered i.p. 2.5-3.0 h after lights-on for both days. *Data analysis and statistics:* EEG, EMG and movement signals were used to classify every 2 second epoch into 6 different sleep stages using the algorithm derived for each individual subject from the baseline day recording. Six sleep-wake states were discriminated - Active waking (AW), Passive waking (PW), Light sleep (LS), Deep sleep (DS), Intermediate sleep (IS) and Rapid Eye Movement sleep (REM). Subsets of the six sleep-wake states were also combined to give Waking (PW and AW), Sleep (IS, REM, LS and DS) and NREM (LS and DS). For each treatment group, the median percentage of time spent in each sleep or waking state were calculated in 3 hour time intervals after drug injection. Significance was calculated by means of the two-sided rank sum test (Mann-Whitney test; P < 0.025).

**Results and Conclusion:** Org 34167 dose dependently increased waking and decreased sleep, including NREM sleep, in the first 3-6 hours of the session when compared to treatment with placebo. The percentage of time spent awake and asleep returned to placebo levels during the 6-9 hour period following treatment, albeit there was a small increase in NREM sleep with Org 34167. Following the light-dark switch Org 34167 demonstrated an increase in waking comparable to the placebo treated group (see Table 1). Org 34167 increases waking and decreases sleep, an effect also observed with wake promoting drugs.

**Table 1: Effects of Org 34167 on sleep-wake organisation in the rat**

| | | **lights on** | | | **lights off** |
|---|---|---|---|---|---|
| **Org 34167 mg/kg** | **Sleep-wake state** | **0-3h** | **3-6h** | **6-9h** | **9-12h** |
| Placebo | Waking | 31.4 | 18.1 | 23.2 | 45.2 |
| 0.3 | Waking | 45.9 * | 19.6 | 22.1 | 52.7 |
| 1 | Waking | 92.4 * | 35.8 * | 18.8 | 48.2 |
| Placebo | Sleep | 68.6 | 81.9 | 76.8 | 54.8 |
| 0.3 | Sleep | 54.1 * | 80.4 | 77.9 | 47.3 |
| 1 | Sleep | 7.6 * | 64.2 * | 81.2 | 51.8 |
| Placebo | NREM | 55.4 | 67.2 | 57.9 | 39.8 |
| 0.3 | NREM | 46.4 * | 63.5 | 63.8 | 40.9 |
| 1 | NREM | 7.6 * | 59.1 * | 63.9 * | 42.1 |

## Claims

1. Use of an Iₕ channel inhibitor for the manufacture of a medicament for the promotion of wakefulness in a subject in need thereof.

2. Use according to claim 1, wherein the subject is suffering from excessive sleepiness.

3. Use according to claim 2, wherein the excessive sleepiness arises from a sleep disorder.

4. Use according to claim 3, wherein the sleep disorder is obstructive sleep apnea/hypopnea syndrome, insomnia or shift work sleep disorder.

5. Use according to any one of claims 1-4, wherein the Iₕ channel inhibitor is selected from:
1,3,4,5-tetrahydro-7,8-dimethoxy-3-[3-[[2-(3,4-dimethoxyphenyl)ethyl]methylimino]propyl]-2H-3-benzazepin-2-one;
(+)-3-[3-[N-[4,5-dimethoxybenzocyclobutan-1 (S)-ylmethyl]-N-methylamino]propyl]-7,8-dimethoxy-2,3,4,5-tetrahydro-*1H*-3-benzazepin-2-one;
2-[N-allyl-N-(2,6-dichlorophenyl)amino]-2-imidazoline and
(S)-2-(1,2-benzisoxazol-3-yl)-α-2-propenylbenzenemethanamine or a pharmaceutically acceptable salt or solvate thereof.
